(19) Europäisches Patentamt
European Patent Office
Office européen des brevets

(11) **EP 4 749 283 A1**

(12) **EUROPÄISCHE PATENTANMELDUNG**

(43) Veröffentlichungstag:
**27.05.2026 Patentblatt 2026/22**

(21) Anmeldenummer: **24215507.5**

(22) Anmeldetag: **26.11.2024**

(51) Internationale Patentklassifikation (IPC):
**G01N 33/543** (2006.01)

(52) Gemeinsame Patentklassifikation (CPC):
**G01N 33/54306;** G01N 2470/04; G01N 2800/28

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Labor Berlin-Charité Vivantes Services GmbH
13353 Berlin (DE)**

(72) Erfinder:
• **KÖRTVELYESSY, Péter
12205 Berlin-Lichterfelde (DE)**
• **MEISEL, Christian
12683 Berlin (DE)**

(74) Vertreter: **Mathys & Squire
Theatinerstraße 8
80333 München (DE)**

(54) **BESTIMMUNG VON PERIPHERIN**

(57) Die vorliegende Erfindung betrifft ein Verfahren zur qualitativen Bestimmung von Peripherin umfassend das Kombinieren und Inkubieren einer verdünnten Probe mit einem ersten Antikörper-Konjugat umfassend einen an einer festen Phase gebundenen ersten Antikörper und einem zweiten Antikörper-Konjugat umfassend einen biotinylierten zweiten Antikörper, das Entfernen der flüssigen Phase und das Auslösen und Messen einer Farbreaktion. Die Erfindung betrifft weiter eine Quantifizierung von Peripherin in einer Probe mittels Kalibrationskurve, das Erstellen einer Kalibrationskurve sowie ein Kit zur Peripherin-Bestimmung.

FIG. 2

EP 4 749 283 A1

## Beschreibung

### Gebiet der Erfindung

Die vorliegende Erfindung betrifft ein Verfahren zur qualitativen Bestimmung von Peripherin umfassend das Kombinieren und Inkubieren einer verdünnten Probe mit einem ersten Antikörper-Konjugat umfassend einen an einer festen Phase gebundenen ersten Antikörper und einem zweiten Antikörper-Konjugat umfassend einen biotinylierten zweiten Antikörper, das Entfernen der flüssigen Phase und das Auslösen und Messen einer Farbreaktion. Die Erfindung betrifft weiter eine Quantifizierung von Peripherin in einer Probe mittels Kalibrationskurve, das Erstellen einer Kalibrationskurve sowie ein Kit zur Peripherin-Bestimmung.

### Hintergrund der Erfindung

Peripherin ist ein Protein der Zellmatrix von Neuronen. Es sorgt zusammen mit anderen sogenannten Intermediär-Filamenten für die Stabilität der Nervenzelle bzw. von Nervenzellfortsätzen. Peripherin ist bisher nicht außerhalb von Nervenzellen gefunden worden und ist somit ein spezifisch nur in Nervenzellen vorkommendes Protein.

Peripherin befindet sich in jeder Nervenzelle außerhalb des Gehirns, z.B. in Motoneuronen auf der Ebene des Spinalmarks und in den Nervenzellfortsätzen, wie z.B. den Axonen. Im autonomen Nervensystem dient Peripherin neben anderen Matrixproteinen zur Stabilisierung der Nervenzellen bzw. Nervenzellfortsätze. Ein Peripherin II genanntes Eiweiß findet sich in der Retina des Auges. Hier hat es dieselbe Funktion und spielt eine wichtige Rolle bei Erkrankungen der Retina, wie z.B. der Makuladegeneration.

Im Falle einer Schädigung der Nervenzelle wird die Zellmatrix der Nervenzellen und der Nervenzellfortsätze teilweise oder ganz zerstört. In diesem Fall wird Peripherin oder Abbauprodukte davon über das umliegende Gewebe auch in die Blutbahn freigesetzt. Das Ausmaß der Freisetzung von Peripherin und/oder seinen Abbauprodukten ist abhängig vom Ausmaß der Nervenzellschädigung. Das bedeutet, dass die Konzentration von Peripherin und/oder seinen Abbauprodukten in einer Körperflüssigkeit Rückschlüsse auf das Ausmaß der Schädigung der zellulären Matrix des Neurons und der Nervenzellfortsätze erlaubt.

Ein ähnlicher Zusammenhang wurde bereits bei anderen Proteinen, die Bestandteil der Zellmatrix von Neuronen sind, gezeigt. Da Peripherin allerdings nur in Nervenzellen des peripheren und nicht in solchen des zentralen Nervensystems vorkommt, lässt sich auf Grundlage der Bestimmung von Peripherin und/oder seiner Abbauprodukte in Körperflüssigkeiten eine Aussage über das Ausmaß und das Kompartment (peripheres oder zentrales Nervensystem) der Nervenzellschädigung treffen. Erhöhte Konzentrationen von Peripherin und/oder seiner Abbauprodukte in Körperflüssigkeiten treten bei Erkrankungen auf, bei denen es zu einer Schädigung der zellulären Matrix von Neuronen außerhalb des Gehirns, also Nervensystems einschließlich des peripheren und des autonomen Nervensystems, kommt.

Bisher konnten Peripherin und/oder seine Abbauprodukte im Blut und anderen Körperflüssigkeiten für eine medizinische Anwendung nicht zufriedenstellend nachgewiesen werden. Die bisherigen Nachweismethoden mittels ELISA Technik sind nicht sensitiv genug, um Peripherin und/oder seine Abbauprodukte in den normalerweise geringen Konzentrationen im Blut, im Liquor cerebrospinales oder anderen Körperflüssigkeiten präzise und zuverlässig nachzuweisen. Diese Techniken sind daher in der klinischen Routine bisher nicht etabliert.

Es besteht daher weiterhin der Bedarf an einem Medizinprodukt zum Nachweis von Peripherin und/oder seiner Abbauprodukte im Blut oder anderen Körperflüssigkeiten.

Es ist daher Aufgabe der vorliegenden Erfindung, die bisherigen methodischen Nachteile in der Peripherin-Messung zu beheben. Es ist insbesondere Aufgabe der vorliegenden Erfindung ein sensitives Verfahren zum Nachweis von Peripherin und/oder seiner Abbauprodukte im Blut oder anderen Körperflüssigkeiten bereitzustellen. Es ist insbesondere Aufgabe der vorliegenden Erfindung ein zuverlässiges Verfahren zum Nachweis von Peripherin und/oder seiner Abbauprodukte im Blut oder anderen Körperflüssigkeiten bereitzustellen. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, dass leicht handhabbar ist, beispielsweise hinsichtlich der Stabilität der eingesetzten Komponenten.

Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches bei einer großen Bandbreite an Peripherin Konzentrationen zuverlässig angewendet werden kann. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches auch bei nichtneurologischen Erkrankungen zuverlässige Perpherin-Konzentrationsbestimmungen ermöglicht. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches sowohl für EDTA-Plasma als auch für Serum zuverlässige Peripherin-Konzentrationsbestimmungen ermöglicht. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches auch bei sehr niedrigen Verdünnungen der Probe zuverlässige Peripherin-Konzentrationsbestimmungen ermöglicht. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches auch bei sehr hohen Verdünnungen der Probe zuverlässige Peripherin-Konzentrationsbestimmungen ermöglicht. Es ist insbesondere Aufgabe der vorliegenden Erfindung ein Verfahren zum Nachweis von Peripherin und/oder seiner Abbauprodukte im Blut oder anderen Körperflüssigkeiten bereitzustellen, welches auch ohne die Verwendung zusätzlicher Konjugate (beispielsweise Helper-beads) zuverlässige Peripherin-Konzentrationsbestimmungen ermöglicht.

## Kurzdarstellung der Erfindung

Die vorliegende Erfindung betrifft ein Verfahren zur qualitativen Bestimmung von Peripherin. Das Verfahren umfasst

a. das Bereitstellen einer Probe;
b. das Verdünnen der Probe mit einem Verdünnungs-Puffer unter Erhalt einer verdünnten Probe;
c. das Kombinieren der verdünnten Probe mit einem ersten Antikörper-Konjugat und einem zweiten Antikörper-Konjugat in einem Reaktionsgefäß unter Erhalt eines ersten Gemisches,
d. das Inkubieren des ersten Gemisches unter Erhalt eines Antikörper-Peripherin-Antikörper-Konjugats, wobei das Peripherin in dem Antikörper-Peripherin-Antikörper-Konjugat zwischen dem ersten Antikörper und dem zweiten Antikörper gebunden ist;
e. das Entfernen der flüssigen Phase und das Waschen der festen Phase mit einem Waschpuffer;
f. das Auslösen einer Farbreaktion durch Zugabe mindestens eines Reporterenzyms und mindestens eines Substrats zu der festen Phase;
g. das Messen der Farbreaktion.

Das erste Antikörper-Konjugat umfasst einen an einer festen Phase gebundenen ersten Antikörper zur Erkennung von Peripherin. Das zweite Antikörper-Konjugat umfasst einen biotinylierten zweiten Antikörper ebenfalls zur Erkennung von Peripherin. Der erste Antikörper ist nicht 8G2, und der zweite Antikörper ist nicht mAb A-3. Das Reporterenzym weist einen Streptavidin-Tag und eine Bindungsstelle zu dem Substrat und keine Bindungsstelle zu dem ersten Antikörper und Peripherin auf. Das Substrat weist eine Bindungsstelle zu dem Reporterenzym und einen Farbstoff-Tag und keine Bindungsstelle zu dem ersten Antikörper, dem zweiten Antikörper und Peripherin auf.

Die vorliegende Erfindung betrifft weiter ein Verfahren zum Erstellen einer Kalibrationskurve für Peripherin. Das Verfahren umfasst das Bestimmen von Peripherin in fünf oder mehr Proben mit bekannter Peripherin-Konzentration gemäß dem oben beschriebenen Verfahren zur qualitativen Bestimmung von Peripherin und das Erstellen einer Kalibrationskurve anhand der Peripherin-Konzentrationen der Proben und der gemessenen Farbreaktion.

Die vorliegende Erfindung betrifft weiter ein Verfahren zur quantitativen Bestimmung von Peripherin. Das Verfahren umfasst

a) das qualitative Bestimmen von Peripherin in einer Probe gemäß dem oben beschriebenen Verfahren zur qualitativen Bestimmung von Peripherin;
b) das Bestimmen einer Kalibrationskurve gemäß dem oben beschriebenen Verfahren zum Erstellen einer Kalibrationskurve für Peripherin; und
c) das Quantifizieren der in Schritt a) gemessenen Farbreaktion durch Vergleich mit der in Schritt b) bestimmten Kalibrationskurve zur Bestimmung der Peripherin-Konzentration in der Probe.

Die vorliegende Erfindung betrifft weiter ein Kit für einen heterogenen Immunoassay zur Bestimmung von Peripherin. Das Kit umfasst

i. ein erstes Antikörper-Konjugat, wobei das erste Antikörper-Konjugat einen an einer festen Phase gebundenen ersten Antikörper zur Erkennung von Peripherin umfasst, und wobei der erste Antikörper nicht 8G2 ist;
ii. ein zweites Antikörper-Konjugat, wobei das zweite Antikörper-Konjugat einen biotinylierten zweiten Antikörper ebenfalls zur Erkennung von Peripherin umfasst, und wobei der zweite Antikörper nicht mAb A-3 ist;
iii. ein Reporterenzym, wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist; und
iv. ein Substrat, wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist.

Das erfindungsgemäße Verfahren löst die oben gestellten Aufgaben. So bietet das erfindungsgemäße Verfahren insbesondere eine hochsensitive Methode, um Peripherin selbst in geringen Konzentrationen zuverlässig in Blut und Körperflüssigkeiten nachzuweisen. Hierfür wird über die definierten Peripherin-spezifischen Antikörper in einem Sandwich-Verfahren das Peripherin gebunden und dann mit Hilfe einer Farbreaktion nachgewiesen. Die Farbreaktion kann durch Vergleich mit einer Kalibrationskurve die Menge an Peripherin in der Probe anzeigen.

Die Anwesenheit und Menge an Peripherin ist ein Marker, um Rückschlüsse auf eine Schädigung des Nervensystems außerhalb vom Gehirn zu ziehen.

Peripherin kann zudem als Biomarker für Krankheiten benutzt werden, für die es bisher noch keine zuverlässig und sensitiv bestimmbaren, bekannten Biomarker gab. Beispiele für solche Krankheiten sind Morbus Crohn, Polyneuropathie und spinale Muskelatrophie.

Generell ist die Möglichkeit, Peripherin zuverlässig und sensitiv bestimmen zu können, in folgenden Bereichen vorteilhaft:

- alle Schädigungen des Spinalmarks, z.B. Multiple Sklerose, spinale Ischämien, Stoffwechselerkrankungen, spinale Tumore, hereditäre Erkrankungen (zum Beispiel: Spinale Muskelatrophie, neurologische Erkrankungen aufgrund von Mutationen in den SCA Genen, Stoffwechselerkrankungen mit Beteiligung des Nervensystems, Spinalparalysen);
- Schädigungen des gastrointestinalen Nervensystems, z.B. Morbus Hirschsprung, Ileus, Colitis ulcerosa, Morbus Crohn, Zöliakie, Pankreatitis, Zystische Fibrose;
- Schädigungen des Auges: z.B. Makuladegeneration und Makuladystrophie, retinale Degeneration und Retinitis Pigmentosa.
- Schädigungen des zentralen Nervensystems (im Falle einer Messung zusammen mit einem Biomarker für eine Schädigung des zentralen Nervensystems kann per Ausschlussverfahren auf eine zentrale Schädigung geschlossen werden, wie z.B. bei dem Apoplex, der Multiplen Sklerose oder der Amyotrophen Lateralsklerose);
- Störungen der Cochlea: z.B. akute Innenohrerkrankungen, Morbus Moniére, Neuronitis verstibularis;
- isolierte Schädigungen des zweiten Motoneurons, z.B. bei der spinalen Muskelatrophie oder der progressiven Muskelatrophie.

### Kurze Beschreibung der Figuren am Beispiel der SIMOA-Technologie:

Fig. 1a-d sind schematische Darstellungen eines Sandwich-ELISA.
Fig. 2a-d sind schematische Darstellungen der SIMOA-Technologie.
Fig. 3 ist eine schematische Darstellung der Bestimmung einer Kalibrationskurve.

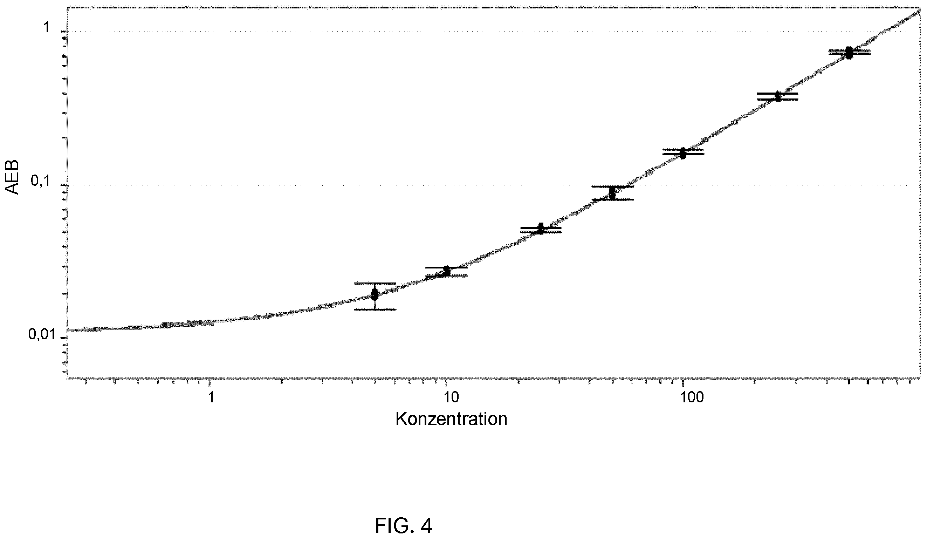

Fig. 4 ist eine beispielhafte Kalibrationskurve

### Detaillierte Beschreibung der Erfindung

Im Rahmen der vorliegenden Erfindung beschreibt der Ausdruck "Peripherin" sowohl Peripherin als auch seine Abbauprodukte.

Im Rahmen der vorliegenden Erfindung beschreibt der Ausdruck "qualitativ" oder "qualitative Bestimmung" die Bestimmung der An- oder Abwesenheit einer Komponente, z.B. Peripherin, in einer Probe. "Quantitativ" oder "quantitative Bestimmung" beschreibt die Bestimmung der Menge oder Konzentration einer Komponente, z.B. Peripherin, in einer Probe.

"Verdünnen" bedeutet im Rahmen der vorliegenden Erfindung die Verringerung der Konzentration einer Komponente (z.B. Peripherin) in einer Probe.

Unter dem Begriff "Antikörper" werden im Rahmen der vorliegenden Erfindung Peripherin-Antikörper verstanden. Die Begriffe "Antikörper", "Antikörper zur Erkennung von Peripherin" und "Peripherin-Antikörper" werden analog verwendet.

Unter dem Begriff "Größe" in Bezug auf Partikel wird jeweils der größte Durchmesser eines Partikels verstanden. Im Rahmen der vorliegenden Erfindung werden die Begriffe "Partikelgröße" und "Größe" analog verwendet. Im Rahmen der vorliegenden Erfindung werden die Begriffe "Partikel" und "Bead" analog verwendet. Partikelgrößen können beispielsweise mittels ISO 13320:2020 bestimmt werden. Im Rahmen der vorliegenden Erfindung bezeichnet die Partikelgröße die mittlere Partikelgröße gemäß ISO 9276-2.

"Annähernd" bedeutet im Rahmen der vorliegenden Erfindung eine Abweichung von weniger als 10 %, z.B. weniger als 5 % oder weniger als 3 %, besonders bevorzugt weniger als 1 %. "Annähernd sphärisch" bedeutet im Rahmen der vorliegenden Erfindung bezogen auf die Form von Partikeln, dass der größte Durchmesser und der kleinste Durchmesser eines Partikels nicht mehr als 10 % (z.B. weniger als 5 % oder weniger als 3 %, besonders bevorzugt weniger als 1 %) voneinander abweichen.

"Raumtemperatur" bezeichnet im Rahmen der vorliegenden Erfindung eine Temperatur im Bereich von 20-25°C.

"Lysepuffer" sind gepufferte Lösungen, die die Bindungen zwischen Proteinen in Proteinaggregate lösen können. Gemäß der vorliegenden Erfindung ist die Verwendung von Lysepuffern vorteilhaft um die Löslichkeit der Proben zu erhöhen.

Die vorliegende Erfindung betrifft ein Verfahren zur qualitativen Bestimmung von Peripherin umfassend

a. Bereitstellen einer Probe;
b. Verdünnen der Probe mit einem Verdünnungs-Puffer unter Erhalt einer verdünnten Probe;
c. Kombinieren der verdünnten Probe mit einem ersten Antikörper-Konjugat und einem zweiten Antikörper-Konjugat in einem Reaktionsgefäß unter Erhalt eines ersten Gemisches,

wobei das erste Antikörper-Konjugat einen an einer festen Phase gebundenen ersten Antikörper zur Erkennung von Peripherin umfasst,
wobei das zweite Antikörper-Konjugat einen biotinylierten zweiten Antikörper ebenfalls zur Erkennung von Peripherin umfasst, und
wobei der erste Antikörper nicht 8G2 ist, und wobei der zweite Antikörper nicht mAb A-3 ist;

d. Inkubieren des ersten Gemisches unter Erhalt eines Antikörper-Peripherin-Antikörper-Konjugats, wobei das Peripherin in dem Antikörper-Peripherin-Antikörper-Konjugat zwischen dem ersten Antikörper und dem zweiten Antikörper gebunden ist;
e. Entfernen der flüssigen Phase und Waschen der festen Phase mit einem Waschpuffer;
f. Auslösen einer Farbreaktion durch Zugabe mindestens eines Reporterenzyms und mindestens eines Substrats zu der festen Phase;

wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist, und
wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist;

g. Messen der Farbreaktion.

Körperflüssigkeiten können als Probe im erfindungsgemäßen Verfahren eingesetzt werden. Bevorzugt sind Blutproben und Liquorproben. Die Proben sind bevorzugt zellfreie Proben, insbesondere zellfreie Liquor-, Serum- und jedwede Plasmaproben. Zellfreie Liquorproben können durch Abnahme einer Liquorprobe, Zentrifugieren und Abtrennen der flüssigen Phase erhalten werden. Serum kann durch Abnahme einer Blutprobe, Initiierung der Gerinnung (z.B. durch Zugabe eines Gerinnungsaktivators oder Verwendung eines Blutröhrchens mit Gerinnungsaktivatoren), Zentrifugation und Abtrennen der flüssigen Phase erhalten werden. Plasma kann durch Abnahme einer Blutprobe, Verhindern der Gerinnung (z.B. durch Zugabe eines Antikoagulans wie EDTA oder Verwendung eines EDTA oder Heparin-Blutröhrchens), Zentrifugation und Abtrennen der flüssigen Phase erhalten werden.

Gemäß Schritt b. wird die Probe mit einem Verdünnungspuffer unter Erhalt einer verdünnten Probe verdünnt. Bevorzugt wird die Probe in einem Verhältnis Probe zu Verdünnungs-Puffer von 1:1-1:100, zum Beispiel 1:2-1:50, besonders bevorzugt 1:3-1:20, verdünnt.

Bevorzugt ist der Verdünnungs-Puffer ein Lysepuffer (z.B. ein Immunokomplex-Lysepuffer). Der pH-Wert des Verdünnungspuffers kann in einem Bereich von 7 bis 8 liegen, zum Beispiel 7,2 bis 7,8, bevorzugt 7,4 bis 7,6.

Gemäß Schritt c. wird die verdünnte Probe mit einem ersten Antikörper-Konjugat (auch Fänger-Antikörper genannt) und einem zweiten Antikörper-Konjugat (auch Detektor-Antikörper genannt) in einem Reaktionsgefäß unter Erhalt eines ersten Gemisches kombiniert.

Dabei kann die Kombination der Komponenten grundsätzlich in beliebiger Reihenfolge erfolgen. Bevorzugt wird die verdünnte Probe zunächst mit dem ersten Antikörper-Konjugat kombiniert, bevor das zweite Antikörper-Konjugat zugegeben wird. Es ist prinzipiell ebenfalls möglich, dass die verdünnte Probe zunächst mit dem zweiten Antikörper-Konjugat kombiniert wird, bevor das erste Antikörper-Konjugat zugegeben wird, oder dass die beiden Antikörper-Konjugate kombiniert werden, bevor die verdünnte Probe zugegeben wird, oder dass alle drei Komponenten werden zumindest teilweise zeitgleich kombiniert.

Die Kombination kann unter Bewegung, beispielsweise Schwenken, des Reaktionsgefäßes erfolgen.

Als Antikörper eignen sich im Rahmen der vorliegenden Erfindung grundsätzlich monoklonalen oder polyklonalen Peripherin-Antikörper, Peripherin-Antikörperfragmente sowie andere Moleküle oder Strukturen, die Peripherin erkennen und binden können. Peripherin-Antikörper sind bekannt und können käuflich erworben werden.

Das erste Antikörper-Konjugat wirkt als Fänger-Antikörper. Es umfasst einen ersten Antikörper, der an einer festen Phase gebunden ist.

Die feste Phase liegt bevorzugt in Form von Partikeln, insbesondere von paramagnetischen Partikeln, vor. Die Partikel können eine Größe von 0,5 bis 10 $\mu$m, z.B. 1 bis 5 $\mu$m, bevorzugt 2 bis 3 $\mu$m aufweisen.

Die Partikel können eine beliebige Form aufweisen. Sie sind bevorzugt sphärisch oder annähernd sphärisch.

Die Partikel sind bevorzugt mit einem Farbstoff markiert, der bei Bestrahlung mit Licht einer bestimmten und bekannten Wellenlänge ein bestimmtes und bekanntes Farbsignal aussendet. Geeignete Partikel sind beispielsweise Simoa 488nm-dyed Singleplex beads (Bestell-Nr. 104006, Quanterix, Billercia, Massachusetts). Sie senden bei Bestrahlung mit einem 488 nm Wellenlänge-Laser ein bestimmtes Farbsignal aus.

Der erste Antikörper ist nicht 8G2. Geeignete, käuflich erwerbliche erste Antikörper sind beispielsweise PRPH Monoclonal Antibody 3B3, (Bestell-Nr. H00005630-M02, Mouse Monoclonal, Nicht biotinyliert, GST-Tag, C-Terminal, IgG2b, Immunogen 374-470, ThermoFisher Scientific, 64293 Darmstadt, Deutschland), Polyclonal Peripherin (PRPH) Antibody, (Bestell-Nr. ABX320907-100UL, Rabbit Polyclonal, Nicht biotinyliert, N-Terminal, IgG, Immunogen 1-260, abbexa, Leiden Bio Science Park, Bargelaan 200, 2333 CW Leiden, Niederlande).

Bevorzugt sind die ersten Antikörper und die Partikel kovalent verknüpft. Die ersten Antikörper und die Partikel können über eine Amidbindung verknüpft sein. Beispielsweise sind die ersten Antikörper über eine primäre Aminogruppe an eine reaktive Carboxylgruppe der Partikel gebunden.

Im ersten Gemisch kann die Menge an erstem Antikörper-Konjugat mindestens der Menge des Peripherins entsprechen. Bevorzugt ist im ersten Gemisch die Menge an erstem Antikörper-Konjugat höher als die Menge Peripherins.

Der zweite Antikörper ist nicht mAb A-3. Geeignete zweite Antikörper sind beispielsweise Peripherin Antibody 8G2, (Bestell-Nr. NB300-138B, Mouse Monoclonal, biotinyliert, IgG1, Bio-Techne GmbH, 65205 Wiesbaden Nordenstadt, Deutschland) und PRPH Monoclonal Antibody 3B3 (Peripherin, Neurofilament 4, NEF4, PRPH1 (Biotin) Clone: [3B3] Mouse Monoclonal, (Bestell-Nr. MBS6143769-0.1), Biotinyliert, GST Tag, C-Terminal, Immunogen 374-470, BIOZOL Diagnostica Vertrieb GmbH, Leipziger Straße 4, 85386 Eching, Deutschland).

Bevorzugt sind der erste Antikörper und der zweite Antikörper verschieden.

Besonders bevorzugt sind der erste Antikörper und der zweite Antikörper verschieden, und der zweite Antikörper ist 8G2.

Gemäß Schritt d. wird das erste Gemisch unter Erhalt eines Antikörper-Peripherin-Antikörper-Konjugats inkubiert. In dem Antikörper-Peripherin-Antikörper-Konjugat ist das Peripherin zwischen dem ersten Antikörper und dem zweiten Antikörper gebunden und entspricht einer Sandwich-Struktur.

Bevorzugt wird das erste Gemisch bei Raumtemperatur inkubiert. Beim Inkubieren kann das erste Gemisch prinzipiell ruhen oder in Bewegung gehalten werden, beispielsweise gerührt, geschüttelt oder geschwenkt werden.

Bevorzugt beträgt die Inkubationszeit des ersten Gemisches 1 min bis 60 min, z.B. 5 bis 50 min oder 10 bis 45 min oder 20 bis 40 min, besonders bevorzugt 35 bis 45 min.

Die Inkubation kann prinzipiell unter verschiedenen Lichtbedingungen gleichermaßen erfolgen, beispielsweise unter Lichtausschluss, bei Tageslicht, Raumlicht oder Bestrahlung. Bevorzugt erfolgt die Inkubation unter Lichtausschluss.

Gemäß Schritt e. wird die flüssige Phase entfernt, und die feste Phase wird mit einem Waschpuffer gewaschen.

Prinzipiell kann die flüssige Phase auf allen dem Fachmann bekannten Wegen entfernt werden. Bevorzugt wird die flüssige Phase mittels pipettierens entfernt. Besonders bevorzugt erfolgt das Entfernen der flüssigen Phase automatisiert mittels automatisierter Pipette.

Der verwendete Waschpuffer ist bevorzugt geeignet, Komponenten zu entfernen, die nicht Teil des Antikörper-Peripherin-Antikörper-Konjugats sind. Beispiele hierfür sind ungebundene Antikörper, ungebundene Komponenten aus der Probe, insbesondere von Peripherin verschiedene Komponenten, und ungebundene Komponenten des Verdünnungspuffers. Bevorzugt ist der Waschpuffer ein Phosphatpuffer. Der bevorzugte pH-Wert des Waschpuffers liegt im Bereich von 7,1 bis 7,7, besonders bevorzugt im Bereich von 7,4 bis 7,5.

Gemäß Schritt f. wird eine Farbreaktion durch Zugabe mindestens eines Reporterenzyms und mindestens eines Substrats zu der festen Phase ausgelöst. Die Farbreaktion ist bevorzugt eine Fluoreszenz- oder Chemilumineszenz-Reaktion, besonders bevorzugt eine Fluoreszenzreaktion.

Das Reporterenzym weist einen Streptavidin-Tag und eine Bindungsstelle zu dem Substrat auf. Über den Streptavidin-Tag kann das Reporterenzym an das Biotin des zweiten Antikörper-Konjugats binden.

Das Reporterenzym weist keine Bindungsstelle zu dem ersten Antikörper und zu Peripherin auf. Damit kann das Reporterenzym selektiv an das zweiten Antikörper-Konjugat des Antikörper-Peripherin-Antikörper-Konjugats binden.

Das Substrat weist eine Bindungsstelle zu dem Reporterenzym und einen Farbstoff-Tag auf. Das Substrat weist keine Bindungsstelle zu dem ersten Antikörper, dem zweiten Antikörper und Peripherin auf.

Bevorzugt wird die Farbreaktion dadurch ausgelöst, dass der Farbstoff des Farbstoff-Tags durch Reaktion mit dem Reporterenzym vom Substrat abgespalten und freigesetzt wird. Der am Substrat gebundene Farbstoff-Tag weist bevorzugt eine andere Farbcharakteristik als der freie Farbstoff des Farbstoff-Tags. Die Farbcharakteristik kann beispielsweise eine Farbe im sichtbaren Bereich, eine Phosphoreszenz, eine Fluoreszenz oder eine Chemilumineszenz sein. Bevorzugt ist die Farbcharakteristik eine Fluoreszenz oder eine Chemilumineszenz, besonders bevorzugt eine Fluoreszenz.

Geeignete Farbstoffe sind beispielsweise Phenoxazin-Farbstoffe. Phenoxazin-Farbstoffe sind beispielsweise in der WO 2010/003970 A2 offenbart. Besonders bevorzugt ist Resorufin.

Das Reporterenzym kann so gewählt sein, dass es das Substrat selektiv vor dem Farbstoff-Tag spaltet, so dass der Farbstoff des Farbstoff-Tags freigesetzt wird. Beispiele für solche Enzym/Substrat-Paare sind Galactosidase und b-Galactopyranosid.

So kann beispielsweise als bevorzugtes Reporterenzym Streptavidin-beta-Galatosidase (SbG) und als bevorzugtes Substrat Resorufin-b-Galactopyranosid (RGP) eingesetzt werden.

Bevorzugt umfasst das Auslösen der Farbreaktion:

f1. die Zugabe des Reporterenzyms und Durchmischen mit der festen Phase; und
f2. Zugabe des Substrats.

Bevorzugt wird die Farbreaktion mittels Simoa-Technik bestimmt.

Simoa steht dabei für "single-molecule array". Bei der Simoa-Technik wird eine Probe (hier umfassend das Antikörper-Peripherin-Antikörper-Konjugat) in Arrays einer Simoa-Scheibe mit einer Vielzahl an Mikrowells gegeben, wobei jedes Mikrowell groß genug für einen Partikel (hier ein Antikörper-Peripherin-Antikörper-Konjugat) ist. Die Farbreaktion kann nach dem Auslösen detektiert werden, indem die Anzahl der Mikrowells mit Farbreaktion bestimmt wird.

Das Ergebnis der Farbreaktion kann in absoluten Werten (also der Gesamtzahl der Mikrowells mit Farbreaktion) oder prozentual (also als prozentualer Anteil der Mikrowells mit Farbreaktion bezogen auf die Gesamtzahl an Mikrowells) angegeben werden. Bevorzugt wird das Ergebnis der Farbreaktion in absoluten Werten angegeben.

Das Vorliegen einer Farbreaktion bedeutet, dass Peripherin in der Probe vorlag.

Eine quantitative Bestimmung des Peripherins erfolgt erfindungsgemäß durch Vergleich der Anzahl der Fluoreszenzen der Probe mit einer Kalibrationskurve, welche basierend auf der fluoreszenten Farbreaktion von Proben mit bekannter Peripherin-Konzentration berechnet wird.

Die vorliegende Erfindung betrifft daher auch ein Verfahren zum Erstellen einer Kalibrationskurve für Peripherin umfassend

i) Bestimmen von Peripherin in fünf oder mehr Proben wie oben beschrieben, wobei die Peripherin-Konzentration jeder der Proben bekannt ist;
ii) Erstellen einer Kalibrationskurve anhand der Peripherin-Konzentrationen der Proben und der gemessenen Farbreaktion.

Zur Erstellung der Kalibrationskurve wird zunächst Peripherin in fünf oder mehr Proben qualitativ bestimmt. Die Bestimmung erfolgt entsprechen dem hierin beschriebenen Verfahren zur qualitativen Bestimmung von Peripherin. Die im Rahmen des Verfahrens zur qualitativen Bestimmung von Peripherin beschriebenen möglichen und bevorzugten Ausführungsformen gelten ebenfalls für die Bestimmung von Peripherin beim Erstellen der Kalibrationskurve.

Bevorzugt wird die Farbreaktion bei der Bestimmung des Peripherins mittels Simoa-Technik wie oben beschrieben bestimmt.

Zur Bestimmung der Kalibrationskurve werden 5 oder mehr Proben verwendet, zu Beispiel 5 bis 15 Proben, bevorzugt 6 bis 8 Proben.

Die Proben können eine Peripherin-Konzentration im Bereich von mehr als 0 pg/mL bis 1.000.000 pg/mL aufweisen, bevorzugt im Bereich von 0,01 pg/mL bis 100.000 pg/mL. Zudem wird bevorzugt eine Referenzprobe, die kein Peripherin enthält, verwendet.

Die Probenkonzentration ist bevorzugt im dem Konzentrationsbereich verteilt, zum Beispiel logarithmisch. Bevorzugt weist mindestens eine der Proben eine Peripherin-Konzentration in einem Bereich von 1 bis 25 pg/mL, z.B. von 5 bis 20 pg/mL auf. Bevorzugt weist mindestens eine der Proben eine Peripherin-Konzentration in einem Bereich von 500 bis 100.000 pg/mL, z.B. von 500 bis 5000 pg/ml auf.

Eine Kalibrationskurve kann durch Auftragen der Ergebnisse in einen Graphen erstellt werden, in dem die X-Achse die Peripherinkonzentration in den Proben und die Y-Achse die Farbreaktion, z.B. in absoluten Werten oder prozentual, bevorzugt in absoluten Werten, bezeichnet.

Bevorzugt wird die Kalibrationskurve anhand einer Fit-Equation der 4PL Regressions Formel

$$Y(x) = D + \frac{A - D}{1 + (\frac{x}{c})^B}$$

bestimmt. Dabei steht

Y für AEB (activated enzyme),
x für die gemessene Konzentration,
D für theoretischer Wert bei einem unendlich hohen Wert,
A für Minimum AEB,
c für Konzentration für das Signal zwischen dem höchsten und niedrigsten Wert und
B für die Steigung der Kurve.

Zur quantitativen Bestimmung von Peripherin wird eine hierin beschriebene qualitative Peripherin-Bestimmung anhand einer hierin beschriebenen Kalibrationskurve quantifiziert.

Die vorliegende Erfindung betrifft daher auch ein Verfahren zur quantitativen Bestimmung von Peripherin umfassend

a) das hierin beschriebene qualitative Bestimmen von Peripherin in einer Probe;
b) das hierin beschriebene Bestimmen einer Kalibrationskurve; und
c) das Quantifizieren der in Schritt a) gemessenen Farbreaktion durch Vergleich mit der in Schritt b) bestimmten Kalibrationskurve.

Die im Rahmen des Verfahrens zur qualitativen Bestimmung von Peripherin und des Verfahrens zum Erstellen einer Kalibrationskurve beschriebenen möglichen und bevorzugten Ausführungsformen gelten ebenfalls für die quantitative Bestimmung von Peripherin.

Bevorzugt werden die Farbreaktion bei der qualitativen Bestimmung von Peripherin und bei der Erstellung der Kalibrationskurve mittels Simoa-Technik bestimmt.

Bevorzugt ist die gemessene Farbreaktion eine Fluoreszenz oder Chemilumineszenz. bevorzugt eine Fluoreszenz.

Die vorliegende Erfindung betrifft auch ein Kit für einen heterogenen Immunoassay zur Bestimmung von Peripherin umfassend

i. ein erstes Antikörper-Konjugat, wobei das erste Antikörper-Konjugat einen an einer festen Phase gebundenen ersten Antikörper zur Erkennung von Peripherin umfasst, und wobei der erste Antikörper nicht 8G2 ist;
ii. ein zweites Antikörper-Konjugat, wobei das zweite Antikörper-Konjugat einen biotinylierten zweiten Antikörper ebenfalls zur Erkennung von Peripherin umfasst, und wobei der zweite Antikörper nicht mAb A-3 ist;
iii. ein Reporterenzym, wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist; und
iv. ein Substrat, wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist.

Die im Rahmen des Verfahrens zur qualitativen Bestimmung von Peripherin beschriebenen möglichen und bevorzugten Ausführungsformen hinsichtlich der Komponenten des Kits gelten ebenfalls für das hier beschriebene Kit.

Bevorzugt umfasst das Kit zudem eine Kalibrationskomponente. Mit Hilfe der Kalibrationskomponente kann eine Kalibrationskurve erstellt werden. Die Kalibrationskomponente umfasst Peripherin in bekannter Menge. Das Peripherin kann in fester oder gelöster Form enthalten sein. Die Kalibrationskomponente ist bevorzugt eine Kalibrationsflüssigkeit, in der das Peripherin in gelöster Form in einem flüssigen Medium, z.B. einem wässrigen Puffer, vorliegt. Liegt das Peripherin in fester Form vor, umfasst das Kit bevorzugt zudem ein flüssiges Medium, z.B. einem wässrigen Puffer, zum Lösen des Peripherins.

Umfasst das Kit eine Kalibrationskomponente, umfasst es bevorzugt zudem eine Anleitung zum Erstellen einer Verdünnungsreihe der Kalibrationskomponente.

Bevorzugt umfasst das Kit zudem einen Verdünnungs-Puffer. Bevorzugt ist der Verdünnungs-Puffer ein Lysepuffer (z.B. ein Immunokomplex-Lysepuffer). Der pH-Wert des Verdünnungspuffers kann in einem Bereich von 7 bis 8 liegen, zum Beispiel 7,2 bis 7,8, bevorzugt 7,4 bis 7,6.

Das erste Antikörper-Konjugat wirkt als Fänger-Antikörper. Es umfasst einen ersten Antikörper, der an einer festen Phase gebunden ist.

Die feste Phase liegt bevorzugt in Form von Partikeln, insbesondere von paramagnetischen Partikeln, vor. Die Partikel können eine Größe von 0,5 bis 10 μm, z.B. 1 bis 5 μm, bevorzugt 2 bis 3 μm aufweisen.

Der erste Antikörper ist nicht 8G2. Geeignete, käuflich erwerbliche erste Antikörper sind beispielsweise PRPH Monoclonal Antibody 3B3, (Bestell-Nr. H00005630-M02, Mouse Monoclonal, Nicht biotinyliert, GST-Tag, C-Terminal, IgG2b, Immunogen 374-470, ThermoFisher Scientific, 64293 Darmstadt, Deutschland), Polyclonal Peripherin (PRPH) Antibody, (Bestell-Nr. ABX320907-100UL, Rabbit Polyclonal, Nicht biotinyliert, N-Terminal, IgG, Immunogen 1-260, abbexa, Leiden Bio Science Park, Bargelaan 200, 2333 CW Leiden, Niederlande).

Bevorzugt sind die ersten Antikörper und die Partikel kovalent verknüpft. Die ersten Antikörper und die Partikel können über eine Amidbindung verknüpft sein. Beispielsweise sind die ersten Antikörper über eine primäre Aminogruppe an eine reaktive Carboxylgruppe der Partikel gebunden.

Im ersten Gemisch kann die Menge an erstem Antikörper-Konjugat mindestens der Menge des Peripherins entsprechen. Bevorzugt ist im ersten Gemisch die Menge an erstem Antikörper-Konjugat höher als die Menge Peripherins.

Der zweite Antikörper ist nicht mAb A-3. Geeignete zweite Antikörper sind beispielsweise Peripherin Antibody 8G2, (Bestell-Nr. NB300-138B, Mouse Monoclonal, biotinyliert, IgG1, Bio-Techne GmbH, 65205 Wiesbaden Nordenstadt, Deutschland) und PRPH Monoclonal Antibody 3B3 (Peripherin, Neurofilament 4, NEF4, PRPH1 (Biotin) Clone: [3B3] Mouse Monoclonal, (Bestell-Nr. MBS6143769-0.1), Biotinyliert, GST Tag, C-Terminal,

Immunogen 374-470, BIOZOL Diagnostica Vertrieb GmbH, Leipziger Straße 4, 85386 Eching, Deutschland)

Bevorzugt sind der erste Antikörper und der zweite Antikörper verschieden.

Besonders bevorzugt sind der erste Antikörper und der zweite Antikörper verschieden, und der zweite Antikörper ist 8G2.

Das Reporterenzym weist einen Streptavidin-Tag und eine Bindungsstelle zu dem Substrat auf. Über den Streptavidin-Tag kann das Reporterenzym an das Biotin des zweiten Antikörper-Konjugats binden.

Das Reporterenzym weist keine Bindungsstelle zu dem ersten Antikörper und zu Peripherin auf. Damit kann das Reporterenzym selektiv an das zweiten Antikörper-Konjugat des Antikörper-Peripherin-Antikörper-Konjugats binden.

Das Substrat weist eine Bindungsstelle zu dem Reporterenzym und einen Farbstoff-Tag auf. Das Substrat weist keine Bindungsstelle zu dem ersten Antikörper, dem zweiten Antikörper und Peripherin auf.

**Beispielhafte Ausführungsformen**

A. Verfahren zur qualitativen Bestimmung von Peripherin umfassend

a. Bereitstellen einer Probe;
b. Verdünnen der Probe mit einem Verdünnungs-Puffer unter Erhalt einer verdünnten Probe;
c. Kombinieren der verdünnten Probe mit einem ersten Antikörper-Konjugat und einem zweiten Antikörper-Konjugat in einem Reaktionsgefäß unter Erhalt eines ersten Gemisches,

wobei das erste Antikörper-Konjugat einen an einer festen Phase gebundenen ersten Antikörper zur Erkennung von Peripherin umfasst,
wobei das zweite Antikörper-Konjugat einen biotinylierten zweiten Antikörper ebenfalls zur Erkennung von Peripherin umfasst, und
wobei der erste Antikörper nicht 8G2 ist, und wobei der zweite Antikörper nicht mAb A-3 ist;

d. Inkubieren des ersten Gemisches unter Erhalt eines Antikörper-Peripherin-Antikörper-Konjugats, wobei das Peripherin in dem Antikörper-Peripherin-Antikörper-Konjugat zwischen dem ersten Antikörper und dem zweiten Antikörper gebunden ist;
e. Entfernen der flüssigen Phase und Waschen der festen Phase mit einem Waschpuffer;
f. Auslösen einer Farbreaktion durch Zugabe mindestens eines Reporterenzyms und mindestens eines Substrats zu der festen Phase;

wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist;
wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist;

g. Messen der Farbreaktion.

B. Das Verfahren gemäß Ausführungsform A oder B, wobei der der erste Antikörper ausgewählt ist aus der Gruppe bestehend aus PRPH Monoclonal Antibody 3B3, Polyclonal Peripherin (PRPH) Antibody (Bestell-Nr. ABX320907-100UL, Rabbit Polyclonal, Nicht biotinyliert, N-Terminal, IgG, Immunogen 1-260, abbexa, Leiden Bio Science Park, Bargelaan 200, 2333 CW Leiden, Niederlande), und wobei der zweite Antikörper ausgewählt ist aus der Gruppe bestehend aus PRPH Monoclonal Antibody 3B3 und Peripherin Antibody 8G2.

C. Das Verfahren gemäß einer der vorstehenden Ausführungsformen, wobei der erste Antikörper und der zweite Antikörper verschieden sind, und wobei der zweite Antikörper 8G2 ist.

D. Das Verfahren gemäß einer der vorstehenden Ausführungsformen, wobei die Probe eine Blutprobe, bevorzugt eine zellfreie Liquorprobe, besonders bevorzugt Humanserum oder Humanplasma ist.

E. Das Verfahren gemäß einer der vorstehenden Ausführungsformen, wobei die Probe in einem Verhältnis Probe zu Verdünnungs-Puffer von 1:1-1:100 verdünnt wird.

F. Das Verfahren gemäß einer der vorstehenden Ausführungsformen, wobei der Verdünnungs-Puffer ein Lysepuffer ist.

G. Das Verfahren gemäß einer der vorstehenden Ausführungsformen, wobei im ersten Gemisch die Menge an erstem Antikörper-Konjugat mindestens der Menge des Peripherins entspricht.

H. Das Verfahren gemäß einer der vorstehenden Ausführungsformen, wobei das erste Antikörper-Konjugat paramagnetische Partikel umfasst, an denen die ersten Antikörper gebunden sind.

I. Das Verfahren gemäß Ausführungsform H, wobei die Partikel eine Größe von 0,5 bis 10 $\mu$m haben.

J. Das Verfahren gemäß einer der vorstehenden Ausführungsformen, wobei der erste Antikörper und der zweite Antikörper verschieden sind.

K. Das Verfahren gemäß einer der vorstehenden Ausführungsformen, wobei das erste Gemisch bei Raumtemperatur inkubiert wird.

L. Das Verfahren gemäß einer der vorstehenden Ausführungsformen, wobei die Inkubationszeit des ersten Gemisches 1 min bis 60 min (bevorzugt 5 bis 50 min, z.B. 10 bis 45 min oder 20 bis 40 min, besonders bevorzugt 35 bis 45 min) beträgt.

M. Das Verfahren gemäß einer der vorstehenden Ausführungsformen, wobei die flüssige Phase mittels Pipettieren entfernt wird.

N. Das Verfahren gemäß einer der vorstehenden Ausführungsformen, wobei der Waschpuffer ein Phosphatpuffer ist.

O. Das Verfahren gemäß einer der vorstehenden Ausführungsformen, wobei das Auslösen der Farbreaktion umfasst:

f1. Zugabe des Reporterenzyms und Durchmischen mit der festen Phase; und
f2. Zugabe des Substrats.

P. Das Verfahren gemäß einer der vorstehenden Ausführungsformen, wobei die Farbreaktion mittels Simoa-Technik bestimmt wird.

Q. Verfahren zum Erstellen einer Kalibrationskurve für Peripherin umfassend

i) Bestimmen von Peripherin in fünf oder mehr Proben gemäß einer der Ausführungsformen A bis P, wobei die Peripherin-Konzentration jeder der Proben bekannt ist;
ii) Erstellen einer Kalibrationskurve anhand der Peripherin-Konzentrationen der Proben und der gemessenen Farbreaktion.

R. Das Verfahren gemäß Ausführungsform Q, wobei die Farbreaktion mittels Simoa-Technik bestimmt wird.

S. Das Verfahren gemäß einer der Ausführungsformen Q oder R, wobei mindestens eine der Proben eine Peripherin-Konzentration in einem Bereich von 1 bis 25 pg/mL aufweist.

T. Das Verfahren gemäß einer der Ausführungsformen Q bis S, wobei mindestens eine der Proben eine Peripherin-Konzentration in einem Bereich von 1.000 bis 100.000 pg/mL aufweist.

U. Verfahren zur quantitativen Bestimmung von Peripherin umfassend

a) qualitatives Bestimmen von Peripherin in einer Probe gemäß einem der Ausführungsformen A bis P;
b) Bestimmen einer Kalibrationskurve gemäß einem der Ausführungsformen Q bis T;
c) Quantifizieren der in Schritt a) gemessenen Farbreaktion durch Vergleich mit der in Schritt b) bestimmten Kalibrationskurve.

V. Das Verfahren gemäß Ausführungsform U, wobei die gemessene Farbreaktion eine Fluoreszenz oder Chemilumineszenz ist.

W. Das Verfahren gemäß Ausführungsform V, wobei die Farbreaktion und die Kalibrationskurve mittels Simoa-Technik bestimmt werden.

X. Kit für einen heterogenen Immunoassay zur Bestimmung von Peripherin umfassend

i. ein erstes Antikörper-Konjugat, wobei das erste Antikörper-Konjugat einen an einer festen Phase gebundenen ersten Antikörper zur Erkennung von Peripherin umfasst, und wobei der erste Antikörper nicht 8G2 ist;
ii. ein zweites Antikörper-Konjugat, wobei das zweite Antikörper-Konjugat einen biotinylierten zweiten Antikörper ebenfalls zur Erkennung von Peripherin umfasst, und wobei der zweite Antikörper nicht mAb A-3 ist;
iii. ein Reporterenzym, wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist; und
iv. ein Substrat, wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist.

Y. Das Kit gemäß Ausführungsform X, ferner umfassend einen Verdünnungs-Puffer (bevorzugt ein Lysepuffer, besonders bevorzugt ein Immunokomplex-Lysepuffer).

Z. Das Kit gemäß einer der Ausführungsformen X oder Y, ferner umfassend eine Kalibrationskomponente, wobei die Kalibrationskomponente Peripherin in bekannte Menge enthält.

AA. Das Kit gemäß einer der Ausführungsformen X bis Z, wobei der erste Antikörper und der zweite Antikörper verschieden sind.

BB. Das Kit gemäß einer der Ausführungsformen X bis AA, wobei das erste Antikörper-Konjugat paramagnetische Partikel umfasst, an denen die ersten Antikörper gebunden sind.

CC. Das Kit gemäß einer der Ausführungsformen X bis BB, wobei die Partikel eine Größe von 0,5 bis 10 $\mu$m haben.

DD. Das Kit gemäß einer der Ausführungsformen AA bis CC, wobei der der erste Antikörper ausgewählt ist aus der Gruppe bestehend aus PRPH Monoclonal Antibody 3B3, Polyclonal Peripherin (PRPH) Antibody (Bestell-Nr. ABX320907-100UL, Rabbit Polyclonal, Nicht biotinyliert, N-Terminal, IgG, Immunogen 1-260, abbexa, Leiden Bio Science Park, Bargelaan 200, 2333 CW Leiden, Niederlande), und wobei der zweite Antikörper ausgewählt ist aus der Gruppe bestehend aus PRPH Monoclonal Antibody 3B3 und Peripherin Antibody 8G2.

EE. Das Kit gemäß einer der Ausführungsformen X bis DD, wobei der erste Antikörper und der zweite Antikörper verschieden sind, und wobei der zweite Antikörper 8G2 ist.

**Beispiele**

**Materialien**

Tabelle 1: Materialien

| | | |
|---|---|---|
| System Buffer 1 | $H_2O$ 90-100%; NaCl 1-5%; $Na_2HPO_4$* 7 $H_2O$ 1-5%; $KH_2PO_4$ 0,01-1%; KCl 0,01-1%; 3(2H)-Isothiazolon-5-chloro-2-methyl mit 2-Methyl-3(2H)-isothiazolon 0,01-1%; Polyoxyethylensorbitan-Monolaurat 0,01-1% | Quanterix Corporation, Billerica, Massachusetts (Bestell-Nr. 100486) |
| System Buffer 2 | $H_2O$ 90-100%; NaCl 0,01-1%; $Na_2HPO_4$ * 7 $H_2O$ 0,01-1%; 3(2H)-Isothiazolon-5-chloro-2-methyl mit 2-Methyl-3(2H)-isothiazolon 0,01-1%; $KH_2PO_4$ 0,01-1%; KCl 0,01-1% | Quanterix Corporation, Billerica, Massachusetts (Bestell-Nr. 100487) |
| Immunkomplex-Lyse Puffer 1L | | GA Generic Assays GmbH, Ludwig-Erhard-Ring 3, 15827 Dahlewitz, Berlin, Deutschland (Bestell-Nr. 16542) |
| Simoa 488nm-dyed Singleplex beads | | Quanterix Corporation, Billerica, Massachusetts (Bestell-Nr. 104006) |
| Bead Wash Buffer | | Quanterix Corporation, Billerica, Massachusetts (Katalog 101335) |
| Bead Conjugation Buffer | | Quanterix Corporation, Billerica, Massachusetts (Katalog 101357) |
| EDC | 1-Ethyl-3-(3-dimethylaminopropyl)carbodiimide | ThermoFisher Scientific, 64293 Darmstadt, Deutschland (Bestell-Nr. 77149) |
| Bead Blocking Buffer | | Quanterix Corporation, Billerica, Massachusetts (Bestell-Nr. 101356) |
| SIMOA Bead Diluent Buffer | Puffer zum Verdünnen der Beads | Quanterix Corporation, Billerica, Massachusetts (Bestell-Nr. 101355) |
| SIMOA Detector/-Sample Diluent | Medium zum Verdünnen der Probe | Quanterix Corporation, Billerica, Massachusetts (Bestell-Nr. 101359) |
| SIMOA Streptavidin-beta-galactosidase (SbG) | SbG-Konzentrat | Quanterix Corporation, Billerica, Massachusetts (Bestell-Nr. 103397) |
| SIMOA SbG Diluent | Medium zum Verdünnen von SbG | Quanterix Corporation, Billerica, Massachusetts (Bestell-Nr. 100376) |
| Resorufin-b-galactopyranoside (RGP) | | Quanterix Corporation, Billerica, Massachusetts (Bestell-Nr. 101736) |
| Rekombinantes humanes Peripherin-Protein | wheat germ (in vitro), N-Terminal, GST Tag | Biozol/Abnova, Taipei, Taiwan (Bestell-Nr. H00005630-P01), |
| Fetales Calf Serum (FCS) | | IGMA Life Sience, (Bestell-Nr. F7524-500ML) |

(fortgesetzt)

| Peripherin Antikör-per (clone 8G2) [Biotin] | Monoklonaler Peripherin-Antikörper aus Mäusen in PBS-Puffer | Novus Biologicals (Bestell-Nr. NB300-138B) |
|---|---|---|
| Bead-gebundene Peripherin Antikörper (clone 3B3) | Monoklonaler Peripherin-Antikörper aus Mäusen in 1x PBS, pH 7,4 | Abanova, Taipei, Taiwan (Bestell-Nr. H00005630-M02) |

**SIMOA-Technologie**

Assays auf Basis der SIMOA (single molecule array) Technologie der Firma Quanterix sind als sensitive Proteinnachweise mit niedriger Nachweisgrenze bekannt. Diese Technologie ermöglicht es auf Einzelmolekül-Ebene einen Proteinnachweis durchzuführen.

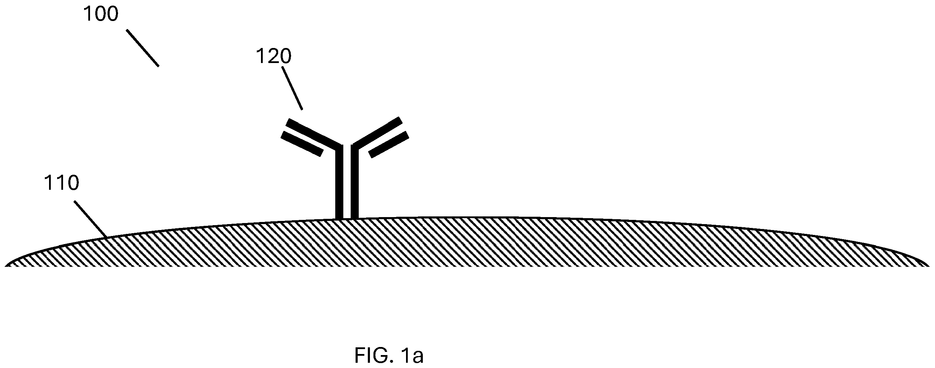

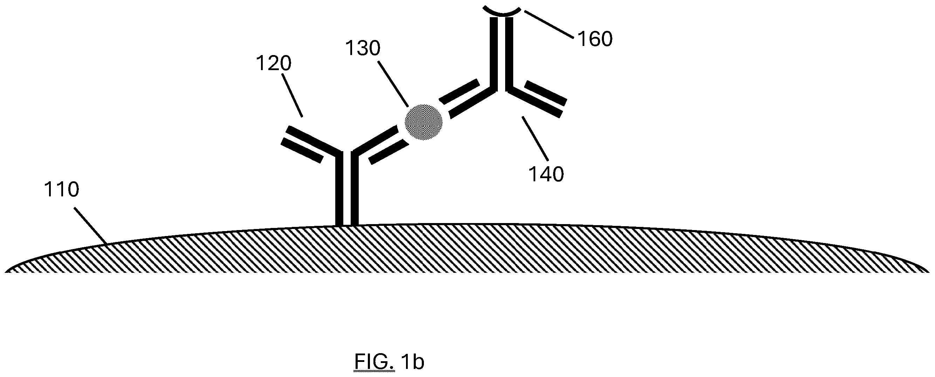

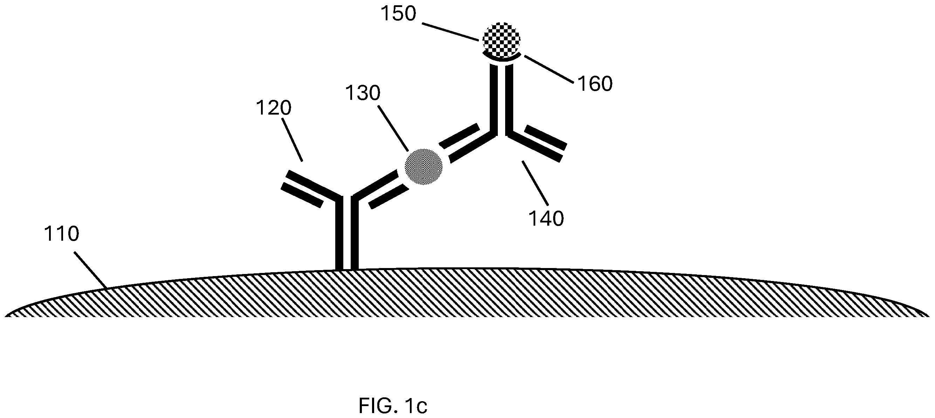

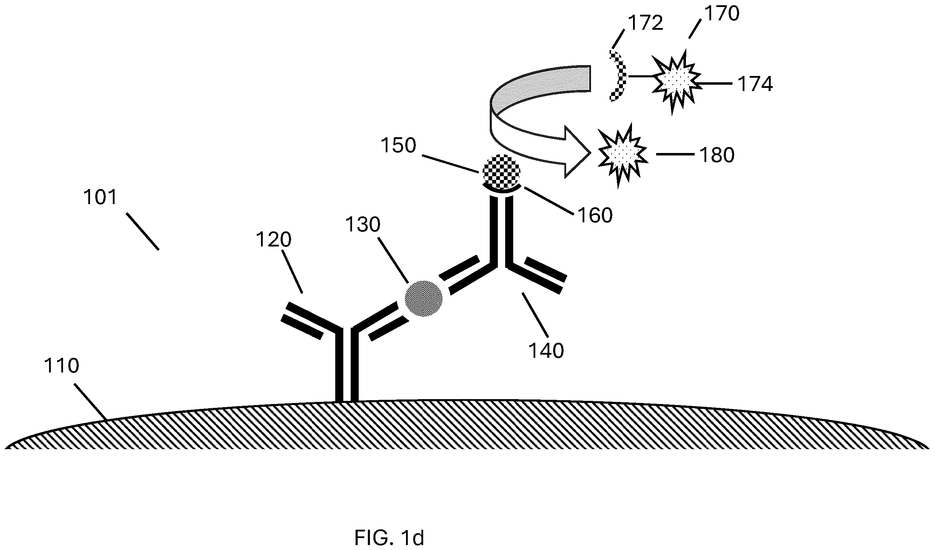

Die Technik beruht auf einem Sandwich-ELISA (enzyme-linked immunosorbent assay), der in FIGs. 1a bis 1d schematisch dargestellt ist. Dabei werden zwei unterschiedliche Antikörper verwendet, welche spezifisch für das jeweilige Zielprotein (130) sind. Ein erster spezifischer Antikörper ("capture-Antikörper" oder "Fänger-Antikörper", 120), der mit einer festen Phase (Beads, 110) verbunden ist, bindet das Zielprotein (130) aus der jeweiligen Matrix. Ein zweiter Antikörper ("detection-Antikörper", 140), der ebenfalls spezifisch das Zielprotein (Peripherin, 130) erkennt ist, bindet an das an der festen Phase (110) über den ersten Antikörper (120) gebundene Zielprotein (130). Der zweite Antikörper (140) ist mit einem Biotin-Tag (160) versehen. Zum Auslösen eines Farbsignals wird zunächst ein mit einem Streptavidin-Tag versehenes Reporterenzym (150) an den Biotin-Tag (160) des zweiten Antikörpers (140) gebunden. Im Anschluss wird ein Substrat (170) zugegeben, dass eine Bindungsstelle (172) zum Reporterenzym (150) und einen Farbstoff-Tag (174) aufweist. Das Reporterenzym kann dann das Substrat zwischen der Bindungsstelle (172) und dem Farbstoff-Tag (174) spalten. Dabei wird der Farbstoff (180) freigesetzt, der detektiert werden kann.

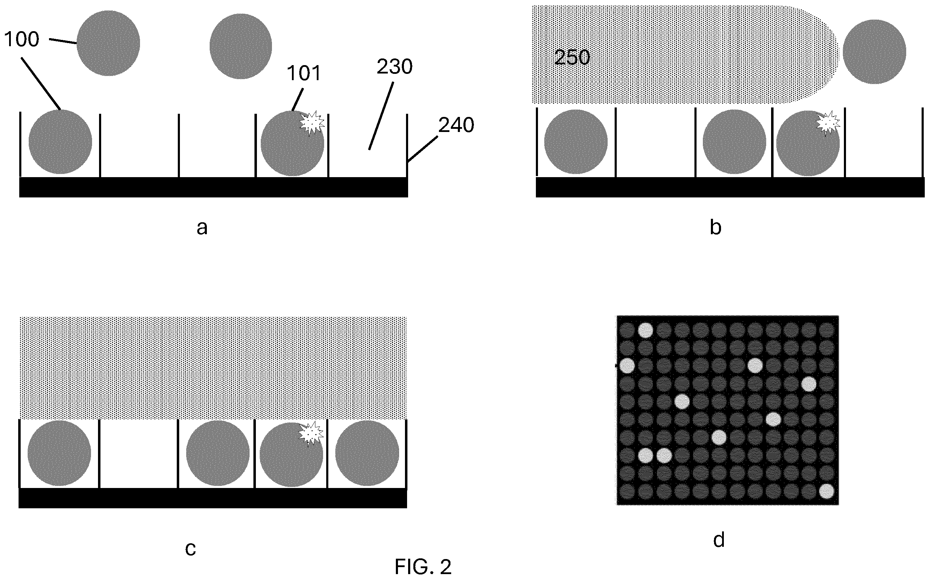

Die SIMOA-Technologie ist eine besonders empfindliche Technologie, die schematisch in FIG. 2 dargestellt ist. Die Reaktionsprobe enthält an Beads (110) gebundene unbeladene erste Antikörper (100), also erste Antikörper, an die keine Zielproteine (130) gebunden haben, und an Beads (110) gebundene beladene erste Antikörper (101), also erste Antikörper, an die Sandwich-artig das Zielprotein (130) und der zweite Antikörper (140) mit Tag (160) gebunden sind. Gemäß FIG. 2a wird die Reaktionsprobe auf eine Mikrotiterplatte (240) mit Wells (230) gegeben, wobei in jedes Well (230) ein an ein Bead gebundener, unbeladener erster Antikörper (100) oder an ein Bead gebundener, beladener erster Antikörper (101) passt. Gemäß FIG. 2b wird ein Öl (250) zum Versiegeln der Wells (230) über die Mikrotiterplatte (240) gegeben. Es wird eine mit Öl (250) versiegelte Mikrotiterplatte (240) erhalten, deren Wells (230) zumindest teilweise mit an ein Bead (110) gebundenen unbeladenen ersten Antikörper (100) oder mit an ein Bead (110) gebundenen beladenen ersten Antikörper (101) gefüllt sind (siehe FIG. 2c). FIG. 2d zeigt, dass durch Auslösen einer Farbreaktion die Wells (230) bestimmt werden können, die beladene erste Antikörper (220) enthalten.

Die Anzahl an Wells, in denen eine Farbreaktion detektiert wird, korreliert mit der Anzahl der gebundenen Zielproteine (130), so dass auf die Konzentration der Zielproteine (130) in der Probe geschlossen werden kann.

**Erstellen einer Verdünnungsreihe für eine Kalibrationskurve**

Die Kalibrationskurve wurde auf Basis einer FCS-Probe ("fetal calf serum") erstellt. Dazu wurde die Serum-Probe für 5 min bei einer Zentrifugalkraft von 10.000 xg zentrifugiert. Die flüssige Phase wurde mittels Pipettieren entnommen.

Die flüssige Phase wurde zunächst mit einem Verdünnungspuffer (Immunkomplex-Lysepuffer 1L) verdünnt (1 Teil FCS-Serum, 3 Teile Verdünnungspuffer) unter Erhalt eines verdünnten FCS-Mediums. Es wurde eine Peripherin-Lösung mit einer Konzentration von 1 pg/mL in Verdünnungspuffer hergestellt.

Anschließend wurden eine Startverdünnung und ein Verdünnungsmedium hergestellt und auf Eis gelagert. Die Zusammensetzungen sind wie folgt:

Tabelle 2: Zusammensetzung Startverdünnung und Verdünnungsmedium

| | Vorverdünnung | Startverdünnung | Verdünnungsmedium |
|---|---|---|---|
| Unverdünntes FCS-Medium | 123,75 µl | 118,75 µl | 425 µL |
| Verdünnungspuffer | 371,25 µl | 356,25 µl | 1275 µL |
| Peripherin-Lösung | 5 µl | | |

(fortgesetzt)

|  | Vorverdünnung | Startverdünnung | Verdünnungsmedium |
|---|---|---|---|
| Vorverdünnung |  | 25 μl |  |

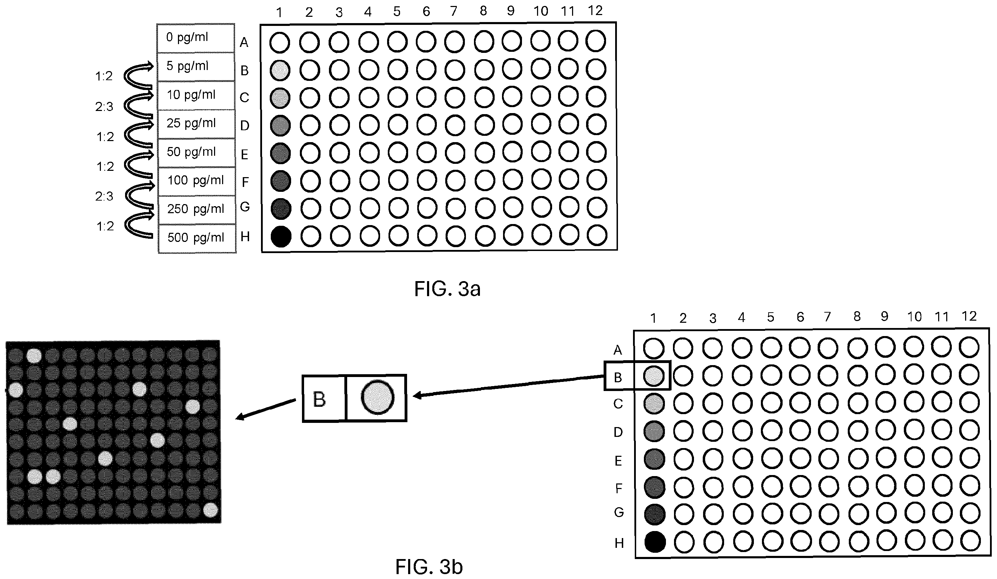

Eine Verdünnungsreihe wurde auf einer SIMOA 96-Wellplatte erstellt (siehe FIG. 3):

i. 400 μL der Startverdünnung wurden in das Well H1 pipettiert.
ii. 200 μL des Verdünnungsmediums und 200 μL Startverdünnung aus dem Well H1 wurden in das Well G1 pipettiert und gut gemischt.
iii. 240 μL des Verdünnungsmediums und 160 μL aus dem Well G1 wurden in das Well F1 pipettiert und gut gemischt.
iv. 200 μL des Verdünnungsmediums und 200 μL aus dem Well F1 wurden in das Well E1 pipettiert und gut gemischt.
v. 200 μL des Verdünnungsmediums und 200 μL aus dem Well E1 wurden in das Well D1 pipettiert und gut gemischt.
vi. 240 μL des Verdünnungsmediums und 160 μL Startverdünnung aus dem Well D1 wurden in das Well C1 pipettiert und gut gemischt.
vii. 200 μL des Verdünnungsmediums und 200 μL aus dem Well C1 wurden in das Well B1 pipettiert und gut gemischt.
viii. 200 μL Verdünnungsmediums wurden in Well A1 pipettiert.

Das Erstellen der Verdünnungsreihe für die Kalibrationskurve ist schematisch in Figur 3a dargestellt. In Reihe 1 der Mikrotiterplatte wird einer Verdünnungsreihe wie oben beschrieben hergestellt. A1 beinhaltet als Referenz das reine Verdünnungsmedium. H1 beinhaltet die Startverdünnung, also die höchste Konzentration an Peripherin. Die abnehmende Menge des Peripherins von H1 bis B1 ist anhand zunehmender Helligkeit der Füllung schematisch dargestellt.
Jeder Proben A1 bis H1 werden wie beschrieben mittels SIMOA-Technik analysiert. Das Ergebnis für die Probe aus Well B1 ist in Figur 3b schematisch dargestellt.

## Herstellung eines ersten Antikörper-Konjugats

Die Herstellung des ersten Antikörper-Konjugats erfolgte mit Hilfe des Simoa Homebrew Assay Starter Kits (Bestell-Nr. 101351, Quanterix, Billercia, Massachusetts). Der gesamte folgende Ablauf wurde auf Eis durchgeführt.
Die Beads wurden 3 mal mit Bead Wash Buffer (Quanterix, (Bestell-Nr. 101335), Quanterix, Billercia, Massachusetts), und danach 3 mal mit Bead Conjugation Buffer gewaschen (Quanterix, (Katalog 101357), Quanterix, Billercia, Massachusetts). Nach dem Waschvorgang wurden 291 μL Bead Conjugation Buffer zu den Beads gegeben.
1-Ethyl-3-(3-dimethylaminopropyl)carbodiimide (EDC) wurde mit Bead Conjugation Buffer auf 10 mg/mL verdünnt. 9 μl des verdünnten EDC wurden zu den Beads in 291 μL Bead Conjugation Buffer gegeben und für 30 min bei 2-8°C in Suspension gemischt, um die Beads zu aktivieren.
80 μg Peripherin-Fängerantikörper wurde mit Bead Conjugation Buffer auf 500 μL aufgefüllt. Mithilfe eines Amicon-Filters aus dem Simoa Homebrew Assay Starter Kit wurden die Antikörper 5 mal mit Bead Conjugation Buffer gewaschen und zentrifugiert (3 mal bei einer Zentrifugalkraft von 14.000 xg für 5 min und 2 mal bei einer Zentrifugalkraft von 1.000 xg für 2 min). Es wurden 300 μL Bead Wash Buffer mit einer Antikörper-Konzentration von 0,2 mg/mL erhalten.
Die aktivierten Beads wurden noch einmal mit 300 μL Bead Wash Buffer gewaschen. Nach Zugabe des 300 μL Antikörper-Gemischs zu den Beads wurde erneut bei 2-8°C unter Schütteln für 2 Stunden inkubiert.
Nach der Inkubation wurde die Suspension 2 mal mit Bead Wash Buffer gewaschen, bevor die Beads-Antikörper-Mischung in 300 μL Bead Blocking Buffer (Quanterix, (Bestell-Nr. 101356), Quanterix, Billercia, Massachusetts) resuspendiert wurde. Nach einer erneuten Inkubation bei Raumtemperatur unter Benutzung einer Schüttelplatte für 45 min wurden die letzten Waschschritte mit Bead Wash Buffer und Bead Diluent (Quanterix, (Bestell-Nr. 101355), Quanterix, Billercia, Massachusetts) durchgeführt, bevor die Beads in Form des ersten Antikörper-Konjugatsin 300 μL Bead Diluent resuspendiert werden.

## Bestimmung von Peripherin

Zur Bestimmung von Peripherin wird die SIMOA-Technik angewandt. Die Durchführung erfolgt automatisiert unter folgenden Bedingungen:

*Geräte und Material*

SIMOA HD-1/HD-X Analyzer (Quanterix Corporation, Billerica, Massachusetts)
SIMOA Pipettenspitzen (Quanterix Corporation, Billerica, Massachusetts)
SIMOA Reagenzgefäße "cuvettes" (Quanterix Corporation, Billerica, Massachusetts)
SIMOA Discs (Quanterix Corporation, Billerica, Massachusetts)
SIMOA 96-Well-Mikrotiterplatten (Quanterix Corporation, Billerica, Massachusetts)
Verdunstungsschutzfolien für 96-Well-Mikrotiterplatten
SIMOA 15-ml Bottles (Quanterix Corporation, Billerica, Massachusetts)

*Material*

SIMOA Bead Diluent Buffer
SIMOA SbG Diluent
SIMOA SbG Concentrate
SIMOA Detector/Sample Diluent
SIMOA RPG Reagent
Immunkomplex-Lyse Buffer
Peripherin-Antikörper (8G2)
Bead-gebundene Peripherin-Antikörper (3B3)
Fetales Kalbserum (FCS)

Eine Liste an Materialien mit näheren Hersteller-Informationen ist auch zu Beginn der Beispiele angegeben.

*Probenvorbereituna*

Mit Hilfe einer von Quanterix zur Verfügung gestellten Exceltabelle zur Berechnung der Volumina werden die 15-ml Bottles mit den jeweiligen Reagenzien gefüllt:

- Peripherin-Antikörper (8G2) mit SIMOA Detector/Sample Diluent,
- SbG-Konzentrat mit SIMOA SbG Diluent,
- Bead-gebundene Peripherin-Antikörper (3B3) mit SIMOA Bead Diluent Buffer.

Die 15-ml Bottles mit den jeweiligen verdünnten Reagenzien werden auf Eis gelagert.
Eine Humanserum-Probe wird für 5 min bei einer Zentrifugalkraft von 10.000 xg zentrifugiert. Die flüssige Phase wird mittels Pipettieren entnommen. Die flüssige Phase wird mit dem Verdünnungspuffer (Immunkomplex-Lysepuffer 1L) verdünnt (1 Teil Serum, 3 Teile Verdünnungspuffer) unter Erhalt einer verdünnten Serum-Probe. Die verdünnte Probe wird bis zur Messung auf Eis gelagert.
Je Messung per Well werden 100 μL der verdünnten Probe benötigt.

*Durchführung*

Die Messung erfolgt auf einem SIMOA HD-1/HD-X Analyzer (Quanterix Corporation, Billerica, Massachusetts) mit integrierter Software.
In der Software werden folgende spezifische Einstellungen zur Durchführung eines benutzerdefinierten Assays zur Peripherin-Bestimmung vorgenommen. Die folgenden Überschriften betreffen die Bezeichnung der jeweiligen Reiter in der Software:

"Reagents"

Tabelle 3: erstes Antikörper-Konjugat

| Angabe in der Software | Bedeutung | Einstellung *(Kommentar)* |
|---|---|---|
| Full Name | Name | *(gewünschter Name, z.B. Peripherin M1 Beads)* |
| Short Name | Abkürzung | *(gewünschte Abkürzung, z.B. Peri M1B)* |

(fortgesetzt)

| Angabe in der Software | Bedeutung | Einstellung *(Kommentar)* |
|---|---|---|
| Type | Typ | Beads |
| Subtype | Subtyp | *(bleibt frei)* |
| Reagent ID | Kennung des Reagenzes | *(bleibt frei)* |
| Total Volume [μL] | Gesamtvolumen | 5000 *(das richtige Volumen wird immer beim Einstellen der Reagenzien vor einer Messung definiert)* |
| Usable Volume [μL] | Verwendbares Volumen | *(passt sich dem Gesamtvolumen an)* |
| On-Board Stability [d:hh:mm] | Haltbarkeit | 6:00:00 |
| Allowed Reagent Lanes | Zulässige Reagenzien bahnen | alle |
| Allowed Substrates | Zulässige Substrate | keine |
| Allowed Positions | Zulässige Positionen | 1-3 |

Tabelle 4: zweites Antikörper-Konjugat

| Angabe in der Software | Bedeutung | Einstellung *(Kommentar)* |
|---|---|---|
| Full Name | Name | *(gewünschter Name, z.B. Peripherin M2 Detektor)* |
| Short Name | Abkürzung | *(gewünschte Abkürzung, z.B. Peri M2D)* |
| Type | Typ | Detektor |
| Subtype | Subtyp | *(bleibt frei)* |
| Reagent ID | Kennung des Reagenzes | *(bleibt frei)* |
| Total Volume [μL] | Gesamtvolumen | 5000 *(das richtige Volumen wird immer beim Einstellen der Reagenzien vor einer Messung definiert)* |
| Usable Volume [μL] | Verwendbares Volumen | *(passt sich dem Gesamtvolumen an)* |
| On-Board Stability [d:hh:mm] | Haltbarkeit | 6:00:00 |
| Allowed Reagent Lanes | Zulässige Reagenzien bahnen | alle |
| Allowed Substrates | Zulässige Substrate | keine |
| Allowed Positions | Zulässige Positionen | 1-6 |

Tabelle 5: SbG

| Angabe in der Software | Bedeutung | Einstellung *(Kommentar)* |
|---|---|---|
| Full Name | Name | *(gewünschter Name, z.B. Peri SbG)* |
| Short Name | Abkürzung | *(gewünschte Abkürzung, z.B. Psbg)* |
| Type | Typ | SbG |
| Subtype | Subtyp | *(bleibt frei)* |
| Reagent ID | Kennung des Reagenzes | *(bleibt frei)* |
| Total Volume [μL] | Gesamtvolumen | 5000 *(das richtige Volumen wird immer beim Einstellen der Reagenzien vor einer Messung definiert)* |
| Usable Volume [μL] | Verwendbares Volumen | *(passt sich dem Gesamtvolumen an)* |
| On-Board Stability [d:hh:mm] | Haltbarkeit | 6:00:00 |
| Allowed Reagent Lanes | Zulässige Reagenzien bahnen | alle |

(fortgesetzt)

| Angabe in der Software | Bedeutung | Einstellung *(Kommentar)* |
|---|---|---|
| Allowed Substrates | Zulässige Substrate | keine |
| Allowed Positions | Zulässige Positionen | 1-3 |

Tabelle 6: Kalibrator

| Angabe in der Software | Bedeutung | Einstellung *(Kommentar)* |
|---|---|---|
| Full Name | Name | *(gewünschter Name, z.B. Peri Calibrator A, usw.)* |
| Short Name | Abkürzung | *(gewünschte Abkürzung, z.B. Peri Cal A, usw.)* |
| Type | Typ | Calibrator |
| Subtype | Subtyp | 0-7 *(0 für Cal. A, 1 für Cal. B)* |
| Reagent ID | Kennung des Reagenzes | *(bleibt frei)* |
| Total Volume [μL] | Gesamtvolumen | 5000 *(das richtige Volumen wird immer beim Einstellen der Reagenzien vor einer Messung definiert)* |
| Usable Volume [μL] | Verwendbares Volumen | *(passt sich dem Gesamtvolumen an)* |
| On-Board Stability [d:hh:mm] | Haltbarkeit | 6:00:00 |
| Allowed Reagent Lanes | Zulässige Reagenzien bahnen | *(nicht einstellbar)* |
| Allowed Substrates | Zulässige Substrate | *(nicht einstellbar)* |
| Allowed Positions | Zulässige Positionen | *(nicht einstellbar)* |

"Overview"

Tabelle 7: allgemeine Einstellung des Assays

| Angabe in der Software | Bedeutung | Einstellung *(Kommentar)* |
|---|---|---|
| Steps | Schritte | 2-step assay neat 50 μL RGP |
| Beads | Erstes Antikörper-Konjugat | (in "Reagents" gespeichertes gewünschtes erstes Antikörper-Konjugat, *z.B. Peri M1B*) |
| Volume Beads [μL]<br>Mini. [μL]<br>Max. [μL] | Volumen Beads<br>Minimales Bead-Volumen<br>Maximales Bead-Volumen | 25<br>10<br>185 |
| Sample / Calibrator | Probe / Kalibrator | *(bleibt frei)* |
| Volumen Sample / Calibrator [μL]<br>Mini. [μL]<br>Max. [μL] | Volumen Probe / Kalibrator<br>Minimales Volumen Probe / Kalibrator<br>Maximales Volumen Probe / Kalibrator | 100<br>10<br>172 |
| Detector | Zweites Antikörper-Konjugat | (in "Reagents" gespeichertes gewünschtes zweites Antikörper-Konjugat, *z.B. Peri M2D*) |
| Volume Detector [μL]<br>Mini. [μL]<br>Max. [μL] | Volumen Detektor<br>Minimales Detektor-Volumen<br>Maximales Detektor-Volumen | 20<br>10<br>185 |
| Incubation time<br>Mini. [cadences] | Inkubationszeit<br>Minimale Kadenzen | 47 cadences (= 47 x 45 s = 5:15 min)<br>1 |

(fortgesetzt)

| Angabe in der Software | Bedeutung | Einstellung (Kommentar) |
|---|---|---|
| Max. [cadences] | Maximale Kadenzen | 1120 |
| SbG | | (in "Reagents" gespeichertes SbG, z.B. Peri SbG) |
| Volume SbG [µL] Mini. [µL] Max. [µL] | Volumen SbG Minimales SbG-Volumen Maximales SbG-Volumen | 100 10 185 |
| Incubation time Mini. [cadences] Max. [cadences] | Inkubationszeit Minimale Kadenzen Maximale Kadenzen | 47 cadences (= 47 x 45 s = 5:15 min) 1 1120 |
| RGP | | |

"Plexes"

Tabelle 8: Einstellungen zur Bestimmung der Kalibrationskurve

| Angabe in der Software | Bedeutung | Einstellung (Kommentar) |
|---|---|---|
| Curve Selection | Kurvenauswahl | Latest |
| Weighting Factor | Gewichtungsfaktor | 1/y2 |
| Fit Algorithm | Anpassungsalgorithmus | 4PL |

Tabelle 9: Messgrößen

| Angabe in der Software | Bedeutung | Einstellung (Kommentar) |
|---|---|---|
| Significant Digits | Relevante Stellen | 12 |
| Precision Digits | Präzisionstellen | 12 |
| Concentration unit | Konzentrationseinheit | pg/µL |

Tabelle 10: Aquisitionskanal

| Angabe in der Software | Bedeutung | Einstellung (Kommentar) |
|---|---|---|
| 750 nm | | Off |
| 700 nm | | Off |
| 647 nm | | Off |
| 488 nm | | L0 |
| Code | | 0001 |

Tabelle 11: weitere Einstellungen

| Angabe in der Software | Bedeutung | Einstellung (Kommentar) |
|---|---|---|
| Calibators | Kalibratoren | 8 Kalibratoren; A-H (gewünschte Abkürzung, z.B. Peri Cal A,Peri Cal 8 usw.) |
| Concentration | Konzentration | 0, 5, 10, 25, 50, 100, 500 |
| Custon Weighing Factor | Benutzerdefinierter Gewichtungsfaktor | 1 (überall) |

**Bezugszeichenliste**

| | |
|---|---|
| 100 | erstes Antikörper-Konjugat |
| 101 | Antikörper-Peripherin-Antikörper-Konjugat mit Reporterenzym und Substrat |
| 110 | feste Phase |
| 120 | erster Antikörper |
| 130 | Zielprotein (z.B. Peripherin) |
| 140 | zweiter Antikörper |
| 150 | Reporterenzym |
| 160 | Biotin-Tag |
| 170 | Substrat |
| 172 | Bindungsstelle zu Reporterenzym |
| 174 | Farbstoff-Tag |
| 180 | Farbstoff |
| 230 | Well |
| 240 | Mikrotiterplatte |
| 250 | Öl |

**Patentansprüche**

1. Verfahren zur qualitativen Bestimmung von Peripherin umfassend

   a. Bereitstellen einer Probe;
   b. Verdünnen der Probe mit einem Verdünnungs-Puffer unter Erhalt einer verdünnten Probe;
   c. Kombinieren der verdünnten Probe mit einem ersten Antikörper-Konjugat und einem zweiten Antikörper-Konjugat in einem Reaktionsgefäß unter Erhalt eines ersten Gemisches,

      wobei das erste Antikörper-Konjugat einen an einer festen Phase gebundenen ersten Antikörper zur Erkennung von Peripherin umfasst,
      wobei das zweite Antikörper-Konjugat einen biotinylierten zweiten Antikörper ebenfalls zur Erkennung von Peripherin umfasst, und
      wobei der erste Antikörper nicht 8G2 ist, und wobei der zweite Antikörper nicht mAb A-3 ist;

   d. Inkubieren des ersten Gemisches unter Erhalt eines Antikörper-Peripherin-Antikörper-Konjugats, wobei das Peripherin in dem Antikörper-Peripherin-Antikörper-Konjugat zwischen dem ersten Antikörper und dem zweiten Antikörper gebunden ist;
   e. Entfernen der flüssigen Phase und Waschen der festen Phase mit einem Waschpuffer;
   f. Auslösen einer Farbreaktion durch Zugabe mindestens eines Reporterenzyms und mindestens eines Substrats zu der festen Phase;

      wobei das Reporterenzym einen Streptavidin-Tag und eine Bindungsstelle zu dem Substrat aufweist, und
      wobei das Reporterenzym keine Bindungsstelle zu dem ersten Antikörper und Peripherin aufweist;
      wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist;

      g. Messen der Farbreaktion.

2. Das Verfahren gemäß Anspruch 1, wobei der der erste Antikörper ausgewählt ist aus der Gruppe bestehend aus PRPH Monoclonal Antibody 3B3, Polyclonal Peripherin (PRPH) Antibody (Bestell-Nr. ABX320907-100UL, Rabbit Polyclonal, Nicht biotinyliert, N-Terminal, IgG, Immunogen 1-260, abbexa, Leiden Bio Science Park, Bargelaan 200, 2333 CW Leiden, Niederlande), und wobei der zweite Antikörper ausgewählt ist aus der Gruppe bestehend aus PRPH Monoclonal Antibody 3B3 und Peripherin Antibody 8G2.

3. Das Verfahren gemäß einem der vorstehenden Ansprüche, wobei der erste Antikörper und der zweite Antikörper verschieden sind, und wobei der zweite Antikörper 8G2 ist.

4. Das Verfahren gemäß einem der vorstehenden Ansprüche, wobei der Verdünnungs-Puffer ein Lysepuffer ist.

5. Das Verfahren gemäß einem der vorstehenden Ansprüche, wobei im ersten Gemisch die Menge an erstem Antikörper-Konjugat mindestens der Menge des Peripherins entspricht.

6. Das Verfahren gemäß einem der vorstehenden Ansprüche, wobei das erste Antikörper-Konjugat paramagnetische Partikel umfasst, an denen die ersten Antikörper gebunden sind.

7. Das Verfahren gemäß Anspruch 6, wobei die Partikel eine Größe von 0,5 bis 10 μm haben.

8. Das Verfahren gemäß einem der vorstehenden Ansprüche, wobei der erste Antikörper und der zweite Antikörper verschieden sind.

9. Das Verfahren gemäß einem der vorstehenden Ansprüche, wobei die Inkubationszeit des ersten Gemisches 1 min bis 60 min beträgt.

10. Das Verfahren gemäß einem der vorstehenden Ansprüche, wobei das Auslösen der Farbreaktion umfasst:

   f1. Zugabe des Reporterenzyms und Durchmischen mit der festen Phase; und
   f2. Zugabe des Substrats.

11. Das Verfahren gemäß einem der vorstehenden Ansprüche, wobei die Farbreaktion mittels Simoa-Technik bestimmt wird.

12. Verfahren zum Erstellen einer Kalibrationskurve für Peripherin umfassend

   i) Bestimmen von Peripherin in fünf oder mehr Proben gemäß einem der Ansprüche 1 bis 9, wobei die Peripherin-Konzentration jeder der Proben bekannt ist;
   ii) Erstellen einer Kalibrationskurve anhand der Peripherin-Konzentrationen der Proben und der gemessenen Farbreaktion.

13. Das Verfahren gemäß Anspruch 12, wobei die Farbreaktion mittels Simoa-Technik bestimmt wird.

14. Das Verfahren gemäß einem der Ansprüche 12 oder 13, wobei mindestens eine der Proben eine Peripherin-Konzentration in einem Bereich von 1 bis 25 pg/mL aufweist, und wobei mindestens eine der Proben eine Peripherin-Konzentration in einem Bereich von 1.000 bis 100.000 pg/mL aufweist.

15. Verfahren zur quantitativen Bestimmung von Peripherin umfassend

   a) qualitatives Bestimmen von Peripherin in einer Probe gemäß einem der Ansprüche 1 bis 11;
   b) Bestimmen einer Kalibrationskurve gemäß einem der Ansprüche 12 bis 14;
   c) Quantifizieren der in Schritt a) gemessenen Farbreaktion durch Vergleich mit der in Schritt b) bestimmten Kalibrationskurve.

16. Kit für einen heterogenen Immunoassay zur Bestimmung von Peripherin umfassend

   i. ein erstes Antikörper-Konjugat, wobei das erste Antikörper-Konjugat einen an einer festen Phase gebundenen ersten Antikörper zur Erkennung von Peripherin umfasst, und wobei der erste Antikörper nicht 8G2 ist;
   ii. ein zweites Antikörper-Konjugat, wobei das zweite Antikörper-Konjugat einen biotinylierten zweiten Antikörper ebenfalls zur Erkennung von Peripherin umfasst, und wobei der zweite Antikörper nicht mAb A-3 ist;
   iii. ein Reporterenzym, wobei das Reporterenzym einen Streptavidin-Tag und eine Bindungsstelle zu dem Substrat aufweist, und wobei das Reporterenzym keine Bindungsstelle zu dem ersten Antikörper und Peripherin aufweist; und
   iv. ein Substrat, wobei das Substrat eine Bindungsstelle zu dem Reporterenzym und einen Farbstoff-Tag aufweist, und wobei das Substrat keine Bindungsstelle zu dem ersten Antikörper, dem zweiten Antikörper und Peripherin aufweist.

17. Das Kit gemäß Anspruch 16, ferner umfassend einen Verdünnungs-Puffer.

**18.** Das Kit gemäß einem der Ansprüche 16 oder 17, ferner umfassend eine Kalibrationskomponente, wobei die Kalibrationskomponente Peripherin in bekannte Menge enthält.

EP 4 749 283 A1

FIG. 1a

FIG. 1b

FIG. 1c

FIG. 1d

FIG. 2

FIG. 3a

FIG. 3b

FIG. 4

**EUROPÄISCHER RECHERCHENBERICHT**

Nummer der Anmeldung

EP 24 21 5507

Europäisches Patentamt
European Patent Office
Office européen des brevets

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Keddie Stephen: "Investigating the pathological mechanism of neuropathy in POEMS syndrome" In: "Thesis", 4. November 2022 (2022-11-04), University College London, XP093268437, Seiten 1-449, Gefunden im Internet: URL:https://discovery.ucl.ac.uk/id/eprint/10155595> * Kapitel 3.1-3.4; Abb 3.3 * ----- | 1-18 | INV. G01N33/543 |
| X | Findlater Joseph: "Peripherin-28 as a Biomarker of ALS: A Methodological Study", , 1. Januar 2010 (2010-01-01), XP093267754, Gefunden im Internet: URL:https://utoronto.scholaris.ca/server/api/core/bitstreams/ab92bdd2-a28a-45c7-b61c-ca28ec3a4e24/content | 1,4,5, 8-10,12, 15-18 | |
| Y | * Abb. 6; Kapitel 3.1, 3.7, 3.10.2, 4.2 * ----- | 1-18 | RECHERCHIERTE SACHGEBIETE (IPC) |
| Y | KEDDIE STEPHEN ET AL: "Peripherin is a biomarker of axonal damage in peripheral nervous system disease", BRAIN, Bd. 146, Nr. 11, 12. Juli 2023 (2023-07-12), Seiten 4562-4573, XP093267501, GB ISSN: 0006-8950, DOI: 10.1093/brain/awad234 Gefunden im Internet: URL:https://academic.oup.com/brain/article-pdf/146/11/4562/52808972/awad234.pdf> * das ganze Dokument * ----- -/-- | 1-18 | G01N |

Der vorliegende Recherchenbericht wurde für alle Patentansprüche erstellt

| Recherchenort | Abschlußdatum der Recherche | Prüfer |
|---|---|---|
| Den Haag | 9. April 2025 | Rosin, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................

& : Mitglied der gleichen Patentfamilie, übereinstimmendes Dokument

EPO FORM 1503 03.82 (P04C03)

Seite 1 von 2

Europäisches Patentamt
European Patent Office
Office européen des brevets

**EUROPÄISCHER RECHERCHENBERICHT**

**Nummer der Anmeldung**

EP 24 21 5507

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | Unknown: "SEF752MU", , 28. Oktober 2023 (2023-10-28), XP093267571, Gefunden im Internet: URL:https://www.cloud-clone.com/manual/ELI SA-Kit-for-Peripherin-(PRPH)-E95752Mu.pdf * das ganze Dokument * ----- | 1-18 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 9. April 2025 | Rosin, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.........................................................................

& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

**In der Beschreibung aufgeführte Patentdokumente**

- WO 2010003970 A2